# EUROPEAN PATENT SPECIFICATION

(11) **EP 1 259 269 B1**
(45) Date of publication and mention of the grant of the patent: **18.06.2008**
(21) Application number: 00909315.4
(22) Date of filing: 03.03.2000
(51) Int. Cl.: A61L 15/22, A61L 15/32, A61L 15/42

(54) **AGENT FOR THE TREATMENT OF WOUNDS**
MITTEL ZUR WUNDBEHANDLUNG
AGENT DE TRAITEMENT DE PLAIES

(43) Date of publication of application: 27.11.2002
(73) Proprietor: Syntacoll AG, 9100 Herisau (CH)
(72) Inventor: SIEGEL, Rolf, D-97076 Würzburg (DE); RUSZCZAK, Zbigniew, D-12557 Berlin (DE); MEHRL, Robert, D-84085 Langweid (DE); JECKLE, Johann, D-93339 Riedenburg (DE); STOLTZ, Michael, D-85540 Haar (DE)
(74) Representative: Böhm, Brigitte
(86) International application number: PCT/EP2000/001905
(87) International publication number: WO 2001/064258

(56) References cited:
- EP-A1- 0 599 589
- DE-A1- 19 542 687
- US-A- 4 060 081
- US-A- 4 265 233
- US-A- 4 660 553
- US-A- 5 770 229
- US-A- 5 928 174

## Description

The invention relates to wound dressings for the treatment of - wounds especially those where not only the upper layer (epidermis), but also the middle and lower layer (dermis) is, at least partially, injured. Such injuries occur, i.e., in second- and third-degree burns, in various different acute and/or chronic skin disorders such as leg ulcers (ulcera crurum) of different origin (arterial and/or venous and/or neuropathic and/or trophic) as well as in pressure ulcers or decubital ulcers. The invention can also be used for the temporary treatment of acute and fresh, both non-surgical and surgical wounds which, for various different reasons, i.e., due to treatment procedure, time, anatomical localization or emergency, the wound cannot be primarily closed by standard surgical procedures or needs to be treated by secondary intention or two-step surgery.

It is known than one can treat wounds of the kind described above by means of fleeces and/or sponges and/or films of natural polymers such as animal collagen, for example, COLLATAMP^{®} and Collatamp^{®}-Fascie, respectively.

It is also known that one can combine natural polymers such as collagen with synthetic polymers such as silicone to create wound dressings.

U.S. Patent 4 060 081, for example, describes preparation of an extremely complex multilayer membrane for treatment of burns, which consists of a collagen sponge containing glycosaminoglycans and mucopolysaccharides and subsequently cross-linked with aldehyde, and a thin silicone layer. This product is commercially available as Integra^{®} Artificial Skin (Integra^{®} Life Sciences, USA). The thin silicone layer is applied to the biological polymer (collagen sponge) in a form of not fully polymerized silicone pre-polymer and is then allowed to polymerize. This is a disadvantage because, during polymerization the acetic acid may be released to the natural polymer layer (collagen sponge); the polymerization process is usually long, up to several days. Moreover, the upper layer of such a dressing (silicone layer) is not air-and water-permeable and this collagen/silicone composite works in fact as an occlusive dressing. This is a disadvantage because an occlusive dressing prevents the exchange of air and fluids so that, i.e., wound fluid may accumulate under the silicone layer. Moreover, due to occlusion, the collagen layer may be degraded more rapidly by wound derived enzymes; this may adversely affect physiological wound healing.

Furthermore, such silicone/collagen wound dressing must be stored in a special container with liquid preservative (in this case a glycerol) which must be removed prior to use. This is a disadvantage because the washing process is time-consuming and for both surgeon and medical staff. EP 0 599 589 is concerned with wound dressings comprising a wound contact layer which may consist of collagen, bonded to a molecular filtration membran covered by an absorbent foam.
The object of the present invention is to create a novel wound dressing which will eliminate disadvantages described above by combination of biological polymers (such as collagen) with pre-manufactured foams of synthetic polymer. This will eliminate the potential risk of diffusion of biologically non-compatible chemical products to natural polymer (i.e. collagen). Moreover, it is an object to provide a possibility to connect both.the synthetic and the natural polymer to a composite product much quicker than is possible for the currently available material.

A further object of the present invention is to create a novel wound dressing in which the biological and the synthetic polymer will be connected to a joint product on a physical-mechanical basis in such a manner that no additional chemical products (such as adhesives) have to be used.

A still further object of the present invention is to create a novel wound dressing in which the biological polymer is designed in such a way that no additional crosslinking procedure is necessary retain a defined its porous or semi-porous structure.

Another object of the present invention is to create a novel wound dressing which will allow both wound fluid and air to penetrate through both the silicone and collagen components.

It is also an object of the present invention to make the final product user-friendly in a way which allows the product to be taken directly from the packing and to be applied directly to the wound surface, without additional preparation or pre-treatment (ready-to-use principle).

Finally, another object of the present invention is to create and to optimize a new method for manufacturing the novel wound dressing which will have a positive economic effect on both manufacturing and treatment costs, if compared to other similar currently marketed products.

The objects of the present invention are achieved by development and manufacturing of a novel agent for the treatment of wounds. This product is composed of two parts, a natural polymer and a synthetic polymer one, and may be obtain by directly applying a (preferentially aqueous) dispersion or/and solution of one or more natural polymers, which may additionally contain substances promoting or accelerating wound healing, on the "wound directed" surface of open- and/or mixed-pore foams of synthetic polymers. Removal of the dispersant or solvents leads to a mechanically stable and physically-based connection (junction) between natural polymer part and synthetic polymer part of the product.

Within the scope of the present invention removal of the dispersant or solvent from the natural polymer can be achieved in a way which allows the natural polymer to have either porous or semi-porous structure or to be a membrane.

According to one embodiment of the present invention in the agent for the treatment of wounds both the biological and the synthetic polymer have an open-pore structure and, according to the invention, the final combination product has a structure which allows the wound to heal physiologically, to enhance the healing ratio, and to exchange fluids, nutrients and gases due to the absence of occlusion.

It is a considerable advantage of the agent for the treatment of wounds according to the invention, that it can be end-sterilized by established techniques (e.g. ethylene oxide, gamma radiation, electron beam, etc.), and hence can be applied to the wound directly from the packing without any preparation or additional treatment prior to use (e.g. washing or thawing).

Moreover, due to the way of industrial manufacturing, art of packing as well as the art of handling and the way of application, the use of the agent developed according to the present invention leads to marked reduction of overall treatment costs.

At present, over 20 different types of collagen are known. Most of them are structurally important for the composition of an extracellular matrix and for the normal function of tissues and organs. Therefore, the natural polymer used for manufacturing of the present agent for healing of wounds, developed according to the present invention, is preferably a collagen, preferably, but not limited to, Type-I, and/or Type-II, and/or Type-III, and/or Tape-VII, and/or Type-IX, and/or a mixture of at least two of them. This collagen may be a natural (native) or/and re-natured collagen obtained from both animal or human tissue, or/and recombinant, or/and transgenic collagen analogous or similar to the animal and/or human collagen, obtained, i.e., by techniques of gene engineering, equivalent or similar.

The use of collagen dispersions for manufacturing collagen-based products is known, see for example, U.S. Patent 4 060 081 or U.S. Patent 4 925 924.

Usually, collagen fibers are isolated from the skins and/or tendons of cattle and/or calves and/or horses by means of known techniques and methods to form a dispersion. The dispersant used according to the invention is usually water, the pH of the dispersion is weakly acidic. The concentration of the collagen is usually less than 4 percent and preferably less than 3 percent of weight. To minimize its potential antigenic properties, the collagen is preferably treated by enzymes to remove non-helical parts of the protein (so-called atelocollagen).

Within the framework of the present invention the natural polymer may consist of a biocompatible and biodegenerable material such as collagen, and may additionally contain polysaccharides, glycosaminoglycans, proteins, cytokines, growth factors (or both natural and/or recombinant and/or transgenic origin), cells or parts of cells, and/or medicaments, i.e., antibiotics.

Within the framework of the present invention the synthetic polymer foam may consist of a non-biodegradable material such as silicone or/and polyurethane or/and polyvinyl alcohol, or/and a biodegradable material such a hyaluronic acid.

Open- and/or mixed-pore foams of synthetic polymers are also known, e.g., foams of polyvinyl alcohol, polyurethane or, preferably, silicone elastomer. These foams are available in various different designs making them suitable for wound treatment. In example, they may be perforated to stream out the wound secretion, may contain holes of various diameter, and, according to the indication of use, may have different dimensions.

The connection between the synthetic polymer, preferably a silicone foam, and the natural polymer, preferably a collagen, may be established as follow: a respective amount of the dispersion or solution of the natural polymer is poured into appropriate forms (e.g. dishes of various sizes and dimensions); a foam of a synthetic polymer (preferably an open-pore silicone foam which can be manufactured, for example, according to DE-A 195 42 687 and DE-A 197 29 227) is then placed on the surface of such dispersion (solution) layer. Fibers of the natural polymer penetrate (due to differences in osmolarity and viscosity of both products) into the pores of the synthetic polymer foam. After removal of the dispersant (for example, by means of drying) both structures interlock so as to form a connection of mechanical-physical nature. The porous structure of the natural polymer is stabilized by pertinent drying processes. As a drying method, for example, freeze-drying or air-drying may be employed. Depending on the type of drying method used, the natural polymer may be formed to a sponge or to a membrane (fascia). The porosity of the natural polymer - in the form of a sponge - can be determined by varying the drying process and/or the polymer concentration or/and by adding salts (preferably sodium, calcium or potassium salts) or/and other biologically acceptable solutions and/or substances; the conditions and measures suitable therefor are known to a skilled artisan.

Alternatively, an (aqueous) dispersion or solution of the natural polymer (preferably collagen) may be applied onto the wound bed directed surface of synthetic polymer (preferably silicon) foams by pouring the dispersion onto the foam surface and then evenly spreading it (preferably by means of a ductor blade). The concentration of the applied dispersion or solution (in grams of dispersed material per cm² of foam surface) can be varied in broad ranges, in respect to the type of dispersion, the type of foam material, its porosity, and the method used to remove the dispersant or solvent.

For example, in the case of using commercially available open-pore silicone foams, 3-Si-Wundauflage^{®}, the use of a 0.3 to 4.0 wt.-% collagen dispersion in water was proved successful.

The methods to subsequently remove the dispersant from the natural polymer solution are known. For example, the dispersion may be allowed to dry out (under reduced pressure and/or increased temperature), yielding tightly adhering sponges (or films) of natural polymer (preferably a collagen) on the surface of the foam of synthetic polymer (preferably silicone). Such natural polymer sponges which adhere to the foam may be obtained by contacting (preferable aqueous) dispersion of the natural polymer which may also contain an easily volatile solvent, e.g. ethanol, and/or an inert gas and/or ice crystals, with the surface and structure of the synthetic polymer foam so that these products combine physically and mechanically by incorporation of natural polymer (or fibers into synthetic polymer. The dispersant (together with pore-forming substances, if added) is then removed, preferably by freeze-drying. To increase the mechanical strength and/or the stability of the natural polymer the final product may additionally be treated hydrothermally or dehydrothermally.

By proceeding like this, the natural polymer compound (preferably collagen fibers) form a physical/mechanical junction with the synthetic polymer (preferably silicone foam) in a way in which the natural polymer components (e.g. collagen fibers) are mechanically anchored to the pores of the synthetic polymer (e.g. silicone foam). After being applied to the wound (according to the indication and intention), the natural polymer (e.g. collagen) will be moistured by blood and/or wound fluids, degraded and/or restructured due to biologically active substances and cells ingrowing from the wound bed and wound borders, which lead to the detachment or separation of the natural polymer part (preferably collagen) from the synthetic polymer (e.g. silicone foam). The natural polymer (preferably collagen) may be, at least partially, incorporated into the wound, and so directly participate in the healing process.

The natural polymer (preferably collagen fibers) stimulates wound healing, provides a natural matrix for the formation of a granulation tissue, accelerates angiogenesis and neovascularization and prevents non-physiological scarring and cicatrising, respectively.

The synthetic polymer (preferably silicone foam) mechanically stabilizes the natural polymer and forms a non-adhesive cover surface of the presently developed wound healing agent. This surface is, at least partially, permeable to air and moisture, the permeability may be controlled by manufacturing process. The synthetic polymer can be easily removed from the surface of the wound due to the natural separation of the natural polymer taking place during the healing process (see above). Due to the open-pore and non adhesive structure of the synthetic polymer used here, the negative properties of actually available synthetic wound dressings such as occlusion or insufficient exchange of air and fluids, as for example by polyurethanes, hydrocolloids, silicone films, as well as possible ingrowth of the dressing into the wound bed, have been avoided.

The present invention allows, for example, to combine known and commercially available natural polymer (preferably a collagen) products such as COLLATAMP^{®}-Fascie (film) and/or COLLATAMP^{®} (sponge) with gas- and water vapor-permeable synthetic polymer (preferably silicon) foams. This improves mechanical stability and strength of the natural polymer, handling of the product, and gives the wound markedly improved protection against exogenous influences (e.g. bacteria, loss of fluids, drying or hyperhydration, temperature changes, etc.). Moreover, this increases the applicability of the products.

In a preferred embodiment the agent for the treatment of wounds manufactured according to the invention, is packed in a standard suitable packaging and end-sterilized by ethylene oxide gassing, gamma radiation, electron beam radiation or by any other suitable sterilization method.

A further subject matter of the present invention is a method for preparing and manufacturing the agent for the treatment of wounds according to the invention, which has been described in detail in the specification above.

### Examples

### Example 1

### Preparation of a collagen sponge/silicone foam composite

50 ml of a 0.56% aqueous collagen dispersion (manufactured by SYNTACOLL AG, Herisau, Switzerland) are poured into an appropriate dish (e.g. 10x10 cm) at room temperature. A 70 x 100 mm open-pore silicone foam (Silcotech AG, Stein am Rhein, Switzerland, known as 3-Si-Wund-auflage^{®}) is placed on the top of the dispersion.

The junction between the bioinert silicone foam and the collagen is formed by removing water, which serves as a dispersant for the natural polymer, by subjecting this arrangement to an air stream and/or controlled negative pressure which allows the natural polymer to dry out.

Described in detail, the natural polymer / synthetic polymer arrangement (i.e. collagen dispersion covered by an open-porous silicon foam, see above) is first continuously frozen in a freezing chamber to about -40°C using a defined temperature gradient, i.e., about 10°C per hour. This allows formation of ice crystals of only preferred and defined dimensions. Thereupon, a negative pressure of about 0.1 mbar is generated in the freezing chamber. The freezing chamber is aerated thereby so as to guarantee a continuous air stream. These conditions ensure controlled sublimation of the dispersant. At the end of the freezing/drying process the chamber is heated stepwise up to room temperature. This method is known for preparation of collagen sponges, and can be performed, for example, in an industrial freeze-drier.

This manufacturing process leads to a collagen/silicone composite, wherein the collagen has the structure of a sponge having desired porosity. The physical properties of the silicone foam are not affected thereby. Since the collagen dispersion penetrates into the pores of the silicone foam, both structures are interlocked on a mechanical-physical basis.

### Example 2

Preparation of a collagen fascia/silicone foam composite

For obtaining a membrane (fascia) of a natural polymer (preferably collagen), adhering to a bioinert foam (prepared from a synthetic polymer, preferably silicone) the water serving as the dispersant of the natural polymer dispersion (e.g. 0.8 wt.-% collagen dispersion) is removed by drying the latter at room temperature and under permanent and controlled air stream without having been previously frozen and/or subjected to the negative pressure. The dispersent completely sublimes thereby. This leads to a collagen/silicone composite, wherein the collagen has the structure of a transparent membrane.

The process of preparing collagen membranes is known and can be carried out without problems in an adequately aerated and heated chamber.

### Example 3

### Preparation of a collagen sponge/silicone foam composite

The 70 x 100 mm, open-porous synthetic polymer (e.g. silicon foam known as 3-Si-Wund-auflage^{®} and manufactured by Silcotech AG, Stein am Rhein, Switzerland) is placed on the bottom of an appropriate dish (e.g. 10x10 cm) at room temperature. 50 ml of a 0.56% dispersion of a natural polymer (e.g. collagen dispersion) in water (manufactured by SYNTACOLL AG, Herisau, Switzerland) are is poured onto this open-pore foam of a synthetic polymer and spread evenly by means of a ductor blade.

The junction between the bioinert silicone foam and the collagen is formed by removing water, which serves as a dispersant for the natural polymer, by subjecting this arrangement to an air stream and/or controlled negative pressure which allows the natural polymer to dry out.

Described in detail, the construct contained from an open-pore silicon foam with the collagen dispersion on the top is first continuously frozen in a freezing chamber to about -40°C using a defined temperature gradient, i.e., about 10°C per hour. This allows formation of ice crystals of only preferred and defined dimensions. Thereupon, a negative pressure of about 0.1 mbar is generated in the freezing chamber. The freezing chamber is aerated thereby so as to guarantee a continuous air stream. These conditions ensure controlled sublimation of the dispersant. At the end of the freezing/drying process the chamber is heated stepwise up to room temperature. This method is known for preparation of collagen sponges, and can be performed, for example, in an industrial freeze-drier.

Said manufacturing process yields a collagen/silicone composite, wherein the collagen has the structure of a sponge having the desired porosity. The physical properties of the silicone foam are not affected thereby. By the collagen dispersion penetrating into the pores of the silicone foam both structures are interlocked on a mechanical/physical basis.

The finished products prepared according to the examples are packed and sterilized by known and proven methods; with regard to sterilization, for example, gassing with ethylene oxide or sterilization with ionizing rays (e.g. gamma irradiation or electron beam) are carried out.

## Claims

1. A non-occlusive composite wound dressing comprising (1) a natural polymer comprised of fibers of one or more natural polymers, and (2) a synthetic polymer foam having an open or mixed pore structure, a surface of the synthetic polymer foam contacting said natural polymer and being adhered to said natural polymer by physical-mechanical interlocking of said polymer fibers with the pores of said synthetic polymer foam surface.

2. A non-occlusive dressing according to claim 1, wherein said natural polymer consists essentially of collagen fibers of animal or human origin or of recombinant and/or transgenic origin.

3. A non-occlusive dressing according to anyone of claims 1 to 2, wherein said natural polymer comprises a porous collagen sponge or a collagen membrane.

4. A non-occlusive dressing according to anyone of the preceding claims, wherein said synthetic polymer foam comprises a non-biodegradable polymer selected from the group consisting of silicone, polyurethane and/or polyvinyl alcohol, and preferably is a silicone polymer.

5. A non-occlusive dressing according to anyone of claims 1 to 4, wherein said synthetic polymer foam comprises a biodegradable polymer.

6. A non-occlusive dressing according to anyone of the preceding claims, that is sterile.

7. A non-occlusive dressing according to anyone of the preceding claims, wherein said natural polymer and said synthetic polymer foam adhere physically to each other and are separable provided that said polymer is in contact with blood and/or wound fluids for a sufficient time for said natural polymer to be moistured and, optionally, at least partially degraded.

8. A non-occlusive dressing according to anyone of the preceding claims, wherein said natural polymer further comprises an additional wound healing agent selected from the group consisting of polysaccharide, glycosaminoglycan, protein, cytokine, growth factor, cells, cell extracts.

9. A non-occlusive dressing according to anyone of the preceding claims, wherein said natural polymer further comprises a medicament, preferably antibiotic.

10. A process for the preparation of a non-occlusive composite wound dressing comprising: (a) contacting a synthetic polymer foam surface comprising an open or mixed pore structure, with a solution and/or dispersion of collagen fibers in a solvent or dispersant; and (b) removing said solvent or dispersant from said collagen fibers under conditions that result in said fibers penetrating into said pores and that form a dried composite of said synthetic polymer foam adhered physically to a layer of said collagen fiber.

11. A process according to claim 10, wherein said solution or dispersion of collagen fiber is applied onto said synthetic polymer foam, or wherein said synthetic polymer foam is applied onto said solution of dispersion of collagen fiber.

12. A process according to claim 10 or 11, further comprising: (c) sterilizing said dried composite; and (d) packaging said sterilized dried composite.

13. A process according to claim 12, wherein said removal comprises freeze-drying and results in a porous collagen fiber sponge.

14. A process according to claim 12, wherein said removal is under conditions that produce a transparent collagen fiber membrane.

15. A non-occlusive composite wound dressing produced in accordance with the process of anyone of claims 10 to 14.

## Patentansprüche

1. Nicht okklusiver Komposit-Wundverband umfassend (1) ein natürliches Polymer, das Fasern von einem oder mehreren natürlichen Polymeren umfasst und (2) einen synthetischen Polymerschaum, der eine offen- oder gemischtporige Struktur hat, wobei eine Oberfläche des synthetischen Polymerschaums das natürliche Polymer berührt und an dem natürlichen Polymer durch physikalisch-mechanische Verzahnung der Polymerfasern mit den Poren der synthetischen Polymerschaumoberfläche anhaftet.

2. Nicht okklusiver Verband nach Anspruch 1, wobei das natürliche Polymer im Wesentlichen aus Kollagenfasern tierischen oder menschlichen Ursprungs oder rekombinanten und/oder transgenen Ursprungs besteht

3. Nicht okklusiver Verband nach einem der Ansprüche 1 oder 2, wobei das natürliche Polymer einen porösen Kollagenschwamm oder eine Kollagenmembran umfasst.

4. Nicht okklusiver Verband nach einem der vorherigen Ansprüche, wobei der synthetische Polymerschaum ein nicht biologisch abbaubares Polymer umfasst, ausgewählt aus der Gruppe bestehend aus Silikon, Polyurethan und/oder Polyvinylalkohol und wobei es bevorzugt ein Silikonpolymer ist

5. Nicht okklusiver Verband nach einem der Ansprüche 1 bis 4, wobei der synthetische Polymerschaum ein biologisch abbaubares Polymer umfasst.

6. Nicht okklusiver Verband nach einem der vorherigen Ansprüche, der steril ist.

7. Nicht okklusiver Verband nach einem der vorherigen Ansprüche, wobei das natürliche Polymer und der synthetische Polymerschaum physikalisch aneinander haften und getrennt werden können, unter der Voraussetzung, dass das Polymer für eine ausreichende Zeit, um angefeuchtet zu werden und, zumindest teilweise abgebaut zu werden, in Kontakt mit Blut und/oder Wundflüssigkeiten ist.

8. Nicht okklusiver Verband nach einem der vorherigen Ansprüche, wobei das natürliche Polymer weiterhin ein zusätzliches Wundheilungsmittel umfasst, ausgewählt aus der Gruppe bestehend aus Polysaccharid, Glykosaminoglykan, Protein, Zytokin, Wachstumsfaktor, Zellen, Zellextrakten.

9. Nicht okklusiver Verband nach einem der vorherigen Ansprüche, wobei das natürliche Polymer weiterhin ein Medikament, bevorzugt ein Antibiotikum, umfasst.

10. Verfahren zur Herstellung eines nicht okklusiven Komposit-Wundverbands umfassend: (a) in Kontakt bringen einer synthetischen Polymerschaumoberfläche, die eine offen- oder gemischtporige Struktur umfasst, mit einer Lösung und/oder Dispersion von Kollagenfasern in einem Lösungs- oder einem Dispergiermittel und (b) Entfernen des Lösungs- oder Dispergiermittels von den Kollagenfasern unter Bedingungen, die dazu führen, dass die Fasern in die Poren eindringen und unter denen ein getrockneter Komposit des synthetischen Polymerschaums gebildet wird, der physikalisch an der Kollagenfaserschicht haftet.

11. Verfahren nach Anspruch 10, wobei die Lösung oder Dispersion von Kollagenfasern auf den synthetischen Polymerschaum aufgebracht wird oder wobei der synthetische Polymerschaum auf die Kollagenfaser-Lösung oder -Dispersion aufgebracht wird.

12. Verfahren gemäß Anspruch 10 oder 11, weiterhin umfassend: (c) Sterilisieren des getrockneten Komposits und (d) Verpacken des sterilisierten getrockneten Komposits.

13. Verfahren nach Anspruch 12, wobei das Entfernen Gefriertrocknen umfasst und zu einem porösen Kollagenfaserschwamm führt.

14. Verfahren nach Anspruch 12, wobei das Entfernen unter Bedingungen geschieht, die eine transparente Kollagenfasermembran herstellen.

15. Nicht okklusiver Komposit-Wundverband hergestellt gemäß dem Verfahren nach einem der Ansprüche 10 bis 14.

## Revendications

1. Pansement composite non occlusif pour des plaies, comprenant (1) un polymère naturel comprenant des fibres d'un ou plusieurs polymères naturels, et (2) une mousse de polymère synthétique ayant une structure de pores ouverts ou mixtes, une surface de la mousse de polymère synthétique étant en contact avec ledit polymère naturel et étant collée audit polymère naturel par l'enchevêtrement physico-mécanique desdites fibres de polymère avec les pores de la surface de ladite mousse de polymère synthétique.

2. Pansement non occlusif selon la revendication 1, dans lequel ledit polymère naturel est constitué essentiellement de fibres de collagène d'origine animale ou humaine ou d'origine recombinée et/ou transgénique.

3. Pansement non occlusif selon l'une quelconque des revendications 1 à 2, dans lequel ledit polymère naturel comprend une éponge de collagène poreuse ou une membrane de collagène.

4. Pansement non occlusif selon l'une quelconque des revendications précédentes, dans lequel ladite mousse de polymère synthétique comprend un polymère non biodégradable choisi dans le groupe constitué par une silicone, un polyuréthane et/ou un poly(alcool vinylique), et est de préférence un polymère de silicone.

5. Pansement non occlusif selon l'une quelconque des revendications 1 à 4, dans lequel ladite mousse de polymère synthétique comprend un polymère biodégradable.

6. Pansement non occlusif selon l'une quelconque des revendications précédentes, qui est stérile.

7. Pansement non occlusif selon l'une quelconque des revendications précédentes, dans lequel ledit polymère naturel et ladite mousse de polymère synthétique adhèrent physiquement entre eux et sont séparables, à condition que ledit polymère soit en contact avec le sang et/ou des liquides de la plaie pendant un temps suffisant pour que ledit polymère naturel soit humidifié et, éventuellement, au moins partiellement dégradé.

8. Pansement non occlusif selon l'une quelconque des revendications précédentes, dans lequel ledit polymère naturel comprend en outre un agent de cicatrisation supplémentaire choisi dans le groupe constitué par un polysaccharide, un glycosaminoglycane, une protéine, une cytokine, un facteur de croissance, des cellules, des extraits de cellules.

9. Pansement non occlusif selon l'une quelconque des revendications précédentes, dans lequel ledit polymère naturel comprend en outre un médicament, de préférence un antibiotique.

10. Procédé de préparation d'un pansement composite non occlusif pour des plaies, comprenant: (a) la mise en contact d'une surface d'une mousse de polymère synthétique comprenant une structure de pores ouverts ou mixtes, avec une solution et/ou une dispersion de fibres de collagène dans un solvant ou un dispersant; et (b) l'élimination dudit solvant ou dispersant desdites fibres de collagènes dans des conditions qui entraînent la pénétration desdites fibres dans lesdits pores et qui forment un composite séché de ladite mousse de polymère synthétique collée physiquement à une couche desdites fibres de collagène.

11. Procédé selon la revendication 10, dans lequel on applique ladite solution ou dispersion de fibres de collagènes sur ladite mousse de polymère synthétique, ou dans lequel on applique ladite mousse de polymère synthétique sur ladite solution ou dispersion de fibres de collagène.

12. Procédé selon la revendication 10 ou 11, comprenant en outre: (c) la stérilisation dudit composite séché; et (d) le conditionnement dudit composite séché stérilisé.

13. Procédé selon la revendication 12, dans lequel ladite élimination comprend une lyophilisation et donne une éponge de fibres de collagène poreuse.

14. Procédé selon la revendication 12, dans lequel ladite élimination s'effectue dans des conditions qui produisent une membrane de fibres de collagène transparente.

15. Pansement composite non occlusif pour des plaies, produit conformément au procédé selon l'une quelconque des revendications 10 à 14.
